# EUROPEAN PATENT APPLICATION

(11) **EP 4 316 546 A1**
(43) Date of publication of application: **07.02.2024**
(21) Application number: 22460057.7
(22) Date of filing: 26.10.2022
(51) Int. Cl.: A61M 1/36, A61M 60/126

(54) **A MEDICAL DEVICE INTENDED FOR USE IN VASCULAR SURGERIES**

(30) Priority: 05.08.2022 PL 44194722
(71) Applicant: Infinity Flow sp. z o.o., 91-134 Lodz (PL)
(72) Inventor: Stepinski, Piotr Wiktor, 91-728 Lódz (PL)
(74) Representative: Dziubinska, Joanna

(57) **Abstract**

A medical device intended for use in vascular surgeries, is characterized in that is in the form of a flexible tube (2) with needles (1) at both ends, with a tap (3) perpendicular to the tube, preferably halfway along its length.

## Description

The subject of the invention is a new and innovative medical device intended for use in vascular surgeries in patients of vascular and cardiac surgery departments to enable surgical interventions which are safer for the patient and at the same time easier and faster.

Various types of cardiac surgery devices are known.

Cervical shunts in the form of a tube ended with balloons are known devices that are inserted into an open artery. Balloons are designed to seal the blood vessels with the pressure in the balloons.

The disadvantage of these solutions is the need to stop the blood flow several times during the surgical intervention in the artery that supplies the brain with blood. Therefore, it causes numerous perioperative and postoperative complications.

The essence of the invention lies in the fact that the medical device intended for use in vascular surgeries is in the form of a flexible tube with needles at both ends, with a tap perpendicular to the tube, preferably halfway along its length.

The solution according to the invention is a blood bypass during surgery. By terminating both ends of the tube with needles, it is possible to insert the device into the artery to seal it around the needles. According to the invention, terminating both sides with needles also allows the bypass of the operated carotid artery without stopping the blood flow both at the stage of inserting the shunt at the beginning of the surgical procedure and during removal of the shunt after the end of the surgical intervention. The length of the flexible tube is known from the state of the art and is each time adjusted to the needs of an operation.

The use of needles in both cannulas in the solution according to the invention makes it easy to puncture this medical device into arteries, easily seal needles inserted into arteries and properly direct the blood flow through the created bypass.

The medical device intended for use in vascular surgeries according to the invention is in the form of a continuous flexible tube (2), the length of which is individually adjusted to the needs of a given patient in a manner known from the state of the art - so that during a surgical procedure the shunt is outside the operating field. The flexible tube (2) is terminated at both of its ends with needles (1) used for puncturing into the arteries. Perpendicular to the tube (2), in the most optimal place in terms of the surgery to be carried out, during which the solution according to the invention is to be used, there is a tap (3) for flushing the device before and during the operation. The tap (3) can be placed anywhere on the tube (2), preferably halfway along its length.

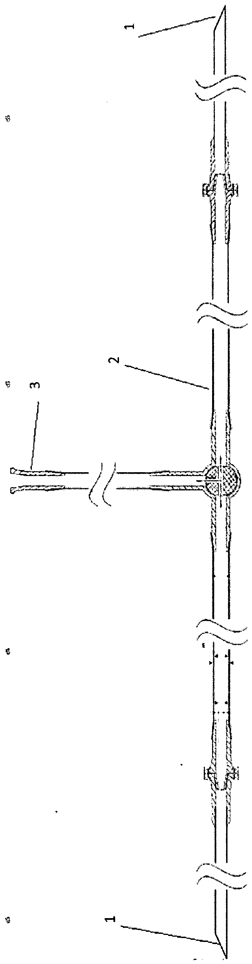

The medical device intended for use in vascular surgeries according to the invention is shown in the attached drawing - Fig. 1.

The solution according to the invention is shown in non-limiting examples of claims.

### Example 1:

The medical device intended for use in vascular surgeries is in the form of a flexible tube (2) terminated at both ends with needles (1) used for puncturing the arteries. The length of the medical device is selected individually to adjust it to the needs of a given patient in a manner known from the state of the art, so that during the surgery the shunt is outside the operating field. Perpendicular to the tube (2), in the middle of its length there is a tap (3) for flushing the device before and during the operation.

### Example 2:

The medical device intended for use in vascular surgeries is in the form of a flexible tube (2) terminated at both ends with needles (1) used for puncturing the arteries. The length of the medical device is selected individually to adjust it to the needs of a given patient in a manner known from the state of the art, so that during the surgery the shunt is outside the operating field. Perpendicular to the tube (2), approximately 1/3 of its length from one of its ends, there is a tap (3) for flushing the device before and during the operation.

## Claims

1. medical device intended for use in vascular surgeries consisting of a flexible tube, having a tap, is **characterized in that** is in the form of a flexible tube (2) with needles (1) at both ends, with a tap (3) perpendicular to the tube, preferably halfway along its length.

## Amended claims

### Amended claims in accordance with Rule 137(2) EPC.

1. A medical device intended for use in vascular surgeries consisting of a flexible tube, having a tap, is **characterized in that** is in the form of a flexible tube (2) with needles (1) at both ends, with a tap (3) perpendicular to the tube, preferably halfway along its length.
